# FASCICULE DE BREVET EUROPEEN

(11) **EP 1 398 624 B1**
(45) Date de publication et mention de la délivrance du brevet: **05.11.2008**
(21) Numéro de dépôt: 02405789.5
(22) Date de dépôt: 11.09.2002
(51) Int. Cl.: G01N 27/12, C07F 11/00, G01N 33/00

(54) **Capteur chimique de gaz et son procédé de fabrication**
Chemischer Gassensor und zugehöriges Herstellungsverfahren
Chemical gas sensor and method of making same

(43) Date de publication de la demande: 17.03.2004
(73) Titulaire: Microchemical Systems S.A., 2035 Corcelles (CH)
(72) Inventeur: Fau, Pierre, 09100 La Tour du Crieu (FR)
(74) Mandataire: GLN

(56) Documents cités:
- WO-A-01/02844
- WO-A-95/35495
- WO-A-99/67181
- US-A- 5 433 971
- TAMAKI J ET AL: "GRAIN SIZE EFFECTS ON TUNGSTEN OXIDE-BASED NITROGEN OXIDES SENSOR" EXTENDED ABSTRACTS, ELECTROCHEMICAL SOCIETY. PRINCETON, NEW JERSEY, US, vol. 93/1, 1993, page 2743 XP000432827 ISSN: 0160-4619
- PATENT ABSTRACTS OF JAPAN vol. 2002, no. 08, 5 août 2002 (2002-08-05) & JP 2002 116178 A (YAZAKI CORP), 19 avril 2002 (2002-04-19)

## Description

La présente invention se rapporte aux capteurs chimiques de gaz. Elle concerne, plus particulièrement, un capteur chimique de gaz à oxyde métallique ainsi que le procédé de réalisation de son élément sensible.

Un capteur de ce type comporte un substrat, un corps de chauffe disposé sur le substrat et un élément sensible en oxyde métallique placé sur le corps de chauffe avec interposition d'une couche électriquement isolante. Son principe repose sur la capacité de l'oxyde métallique, porté à haute température (environ 400°C), à adsorber le gaz ambiant qui modifie ainsi sa résistivité. La variation de résistance de cette couche sensible à une température donnée est représentative de la quantité de gaz à détecter. La sensibilité du capteur est définie comme le rapport de la résistance de la couche sensible en absence du gaz, à sa résistance en présence du gaz.

A ce jour, l'élément sensible de ces capteurs est généralement de l'oxyde d'étain (SnO₂ ou de l'oxyde de tungstène (WO₃). Ils sont principalement destinés à constituer le coeur des systèmes de sécurité de plus en plus utilisés pour la détection, dans l'air ambiant, de gaz nocifs tels que le monoxyde de carbone, les oxydes d'azote, l'ozone, les hydrocarbures,... tant dans le domaine domestique que dans l'industrie, ou pour contrôler l'admission d'air dans l'habitacle des véhicules.

Le document WO 01/02844 décrit un tel capteur et son procédé de fabrication qui garantit une qualité optimale de sa couche sensible car elle est déposée lors de l'ultime étape du procédé et n'est donc pas altérée par les autres étapes du procédé.

Le document EP 1273908 propose également un capteur de gaz à SnO₂ dont la structure permet :
- d'obtenir une température particulièrement homogène du corps de chauffe;
- d'améliorer la longévité des contacts électriques du corps de chauffe;
   et
- de réaliser in situ la stabilisation thermique de l'élément sensible.

Il est connu qu'une couche sensible en oxyde de tungstène (WO₃) offre de meilleurs résultats que le SnO₂ en matière de sensibilité aux gaz oxydants tels que les NOx ou ceux qu'émettent les véhicules à moteur diesel.

Il est déjà connu de utiliser des couches sensibles de WO3 dans des capteurs de NOx dans le document suivant:
TAMAKI J ET AL: "GRAIN SIZE EFFECTS ON TUNGSTEN OXIDE-BASED NITROGEN OXIDES SENSOR" EXTENDED ABSTRACTS, ELECTROCHEMICAL SOCIETY. PRINCETON, NEW JERSEY, US, vol. 93/1, 1993, page 2743 ISSN: 0160-4619

Toutefois, les procédés classiquement utilisés pour déposer du W03 conduisent à l'obtention de couches épaisses, nécessitant des recuits de frittage à des températures supérieures à 900°C pour souder les grains entre eux. Ces grains sont gros (quelques microns) et ont une sensibilité limitée, du fait de la faiblesse de la surface d'échange entre les grains, ce qui oblige à augmenter le volume de l'élément sensible et, par conséquent, sa consommation électrique.

La présente invention a pour but de pallier les inconvénients présentés jusqu'alors par les dépôts de WO₃, tout en combinant les avantages dont dispose la technologie des capteurs chimiques de gaz à SnO₂.

De façon plus précise, l'invention concerne un capteur chimique de gaz à oxyde métallique, du type comportant :
- un cadre délimitant une fenêtre centrale,
- une couche diélectrique déposée sur le cadre et dont la partie en regard de sa fenêtre forme une membrane,
- une couche conductrice, formant corps de chauffe, disposée sur la membrane,
- une couche électriquement isolante recouvrant le corps de chauffe,
- un élément sensible d'oxyde métallique déposé au centre de ladite couche isolante et comportant une couche d'oxyde de tungstène (WO₃) qui se présente sous la forme d'une poudre agglomérée à structure poreuse, et
- deux électrodes disposées au contact dudit élément sensible pour permettre de mesurer sa résistance électrique,

Selon l'invention, un tel capteur est caractérisé en ce que ladite poudre d'oxyde de tungstène est constituée de particules de taille submicronique en forme d'écailles.

L'invention concerne également un procédé pour la réalisation de l'élément sensible en oxyde de tungstène du capteur défini ci-dessus. Il comporte les opérations successives de :
- dissolution de tungstate de sodium hydraté dans de l'eau distillée,
- passage à travers une colonne contenant une résine échangeuse de protons pour obtenir une solution d'acide tungstique,
- évaporation - concentration de cette solution afin d'obtenir, par précipitation, des particules d'oxyde de tungstène hydraté,
- dépôt de ces particules, sous forme d'une goutte, au centre de la couche isolante, et
- recuit du capteur à une température d'environ 300 à 600°C.

D'autres caractéristiques de l'invention ressortiront de la description qui va suivre, faite en regard du dessin annexé, dans lequel :
- la figure 1 est une vue de dessus du capteur,
- la figure 2 est une vue agrandie de sa partie centrale avant le dépôt de l'élément sensible, et

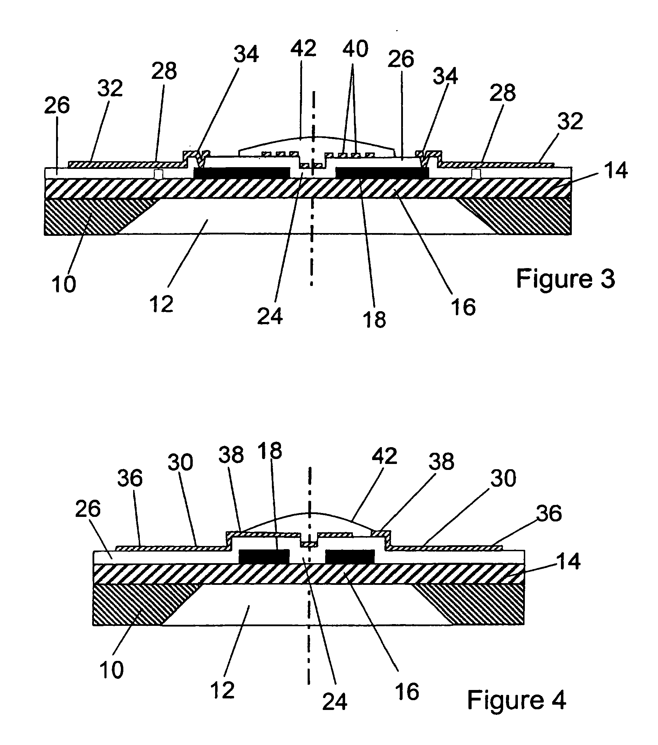

- les figures 3 et 4 le représentent en coupe, respectivement selon XX et YY.

Le dessin montre un cadre rectangulaire 10 en matériau semi-conducteur, tel que le silicium monocristallin, possédant une fenêtre centrale 12, également rectangulaire, dotée de deux axes de symétrie orthogonaux XX et YY. Typiquement, cette fenêtre a une longueur, selon XX, de 1 mm et une largeur, selon YY, de 0.75 mm.

Le cadre 10 est totalement recouvert d'une couche diélectrique en oxynitrure 14 dont la partie en regard de la fenêtre 12 forme une membrane 16. L'épaisseur de cette couche est de l'ordre de 2 µm.

La membrane 16 est, elle-même, recouverte partiellement d'un corps de chauffe 18 formé d'une couche de silicium polycristallin rendu conducteur par dopage, par exemple, au moyen de phosphore. L'épaisseur de cette couche est d'environ 0,40 µm.

Le corps de chauffe 18 a la forme d'un papillon, avec les mêmes axes de symétrie XX et YY que l'ouverture 12 et la membrane 16. Il possède une partie centrale 20 et deux ailes latérales identiques 22.

La partie centrale 20 est rectangulaire. Sa largeur, selon YY, et sa longueur, selon XX, correspondent environ respectivement à la moitié de la largeur et de la longueur de la fenêtre 12. Elle est percée, en son centre, d'un trou carré 24 d'environ 100 µm de côté dont l'utilité apparaîtra plus loin.

Les ailes 22 du corps de chauffe 18 s'étendent, selon XX, en s'élargissant depuis la partie centrale 20 pour se terminer à proximité des bords de la membrane 16.

Le tout est recouvert d'une couche d'isolation 26, par exemple, en oxyde ou nitrure de silicium, sur laquelle reposent, selon XX, deux pistes conductrices 28 et, selon YY, deux pistes conductrices 30.

Une extrémité de chaque piste 28 possède une plage de connexion 32, tandis que son autre extrémité 34 s'étend tout au long de l'extrémité de l'aile 22 du corps de chauffe, qu'elle rejoint en traversant la couche d'isolation 26.

Une extrémité de chaque piste 30 possède également une plage de connexion 36, tandis que son autre extrémité 38 s'étend le long du bord de la partie centrale 20 du corps de chauffe sur une longueur sensiblement inférieure à la longueur de cette partie centrale. Typiquement, dans l'exemple décrit, l'extrémité 38 a une longueur d'environ 300 µm.

Un tel capteur fait l'objet de la demande EP 1273908 déjà citée.

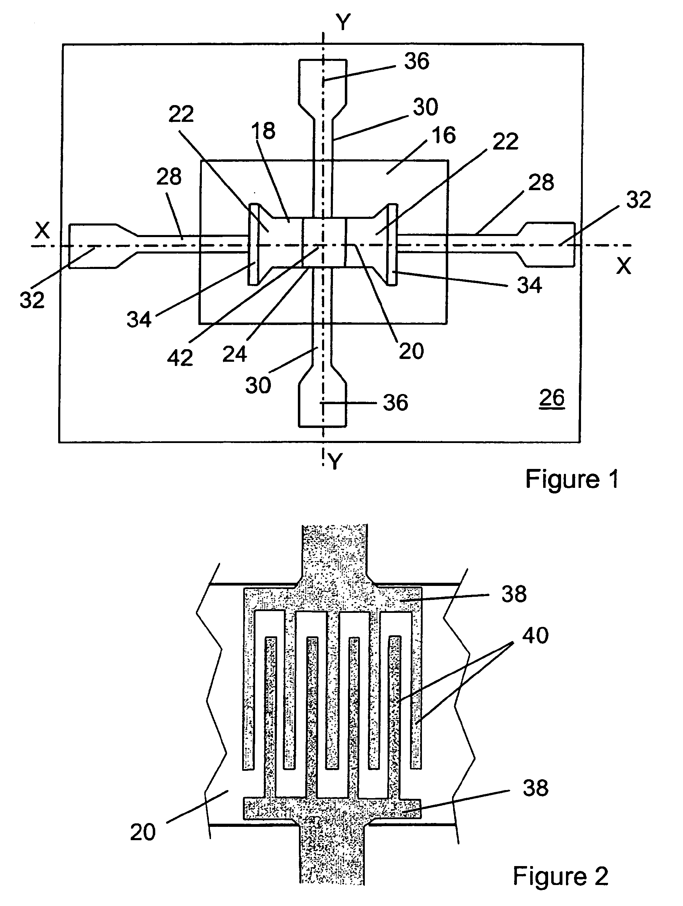

Comme le montre seulement la figure 2, les extrémités 38 des pistes 30 se prolongent, l'une vers l'autre, par un réseau de doigts parallèles 40 qui s'interpénètrent pour constituer une paire d'électrodes interdigitées.

De manière particulièrement avantageuse, les pistes 30, leurs extrémités 38 et leurs doigts 40 sont en platine recouvert d'or.

Finalement, le capteur selon l'invention comporte un élément sensible 42 disposé sur la couche d'isolation 26, au centre de la structure, en forme de goutte solide, de manière à recouvrir les deux réseaux d'électrodes 40. Typiquement, la goutte a un diamètre d'environ 300 µm et une épaisseur au centre de quelques µm.

Selon une caractéristique importante de l'invention, l'élément sensible 42 est constitué d'une couche d'oxyde de tungstène (WO₃) se présentant sous la forme d'une poudre agglomérée à structure poreuse constituée de particules de taille sub-micronique.

Plus précisément, les particules de la couche de WO₃ peuvent présenter des formes diverses: écailles, pavés, aiguilles, ...

On notera que les plages 32 servent à relier le corps de chauffe 18 à une source d'énergie électrique nécessaire à son alimentation, alors que les plages 36 servent à relier le capteur à une électronique de mesure de la résistance de l'élément sensible 42.

Comme mentionné dans le document EP1273908, une telle structure permet, par un effet d'entonnoir, de concentrer les lignes de courant du corps de chauffe 18 sur sa partie centrale 20, percée du trou 24, et donc de porter l'élément sensible 42 à une température remarquablement uniforme.

Selon une autre caractéristique de l'invention, la couche sensible de WO₃ est issue de la précipitation de l'acide tungstique.

La méthode consiste à dissoudre du tungstate de sodium hydraté dans de l'eau distillée puis à faire passer le tout à travers une colonne contenant une résine échangeuse de protons pour obtenir une solution d'acide tungstique.

La précipitation est alors réalisée par évaporation - concentration de cette solution afin d'obtenir des particules d'oxyde de tungstène hydraté d'acide tungstique du type WO₃, xH₂O. Ces particules sont alors déposées, sous forme d'une goutte, au centre de la structure à l'aide d'une micro pipette assurant un contrôle très précis du volume délivré. La phase liquide s'évapore ensuite en quelques secondes à l'air ambiant.

Il ne reste plus alors qu'à effectuer un recuit du capteur à une température d'environ 300 à 600°C, opération qui a pour effet d'éliminer l'eau de constitution et, par cristallisation, de transformer les particules de WO₃, xH₂O en WO₃.

Ainsi est proposé un capteur de gaz mettant pleinement à profit la capacité du WO₃ pour répondre, de manière sélective et sensible, aux gaz oxydants tels que ceux dégagés par les moteurs diesel. En effet, la taille sub-micronique et la forme des particules :
- améliorent fortement, grâce aux grandes surfaces d'échange procurées, la réactivité de l'élément sensible en WO₃,
- facilitent son adhérence au substrat,
- réduisent très fortement les risques de fissures ou défauts dans la couche déposée,
- assurent son excellente résistance mécanique, et
- permettent de réduire sensiblement la taille du capteur.

Le capteur selon l'invention présente, en outre, les principaux avantages suivants :
- facilité de préparation et de dépôt de la couche sensible en WO₃ (pipettage précis, recuit à température peu élevée),
- grâce aux électrodes interdigitées, possibilité de mesurer le signal à un niveau de résistance compatible avec une électronique peu coûteuse,
- grâce aux électrodes de platine recouvertes d'or, amélioration de la sélectivité aux gaz oxydants, de la sensibilité et de la stabilité dans le temps.

## Revendications

1. Capteur chimique de gaz à oxyde métallique comportant :
- un cadre (10) délimitant une fenêtre centrale (12),
- une couche diélectrique (14) déposée sur ledit cadre et dont la partie en regard de sa fenêtre forme une membrane (16),
- une couche conductrice (18), formant corps de chauffe, disposée sur ladite membrane,
- une couche électriquement isolante (26) recouvrant ledit corps de chauffe,
- un élément sensible d'oxyde métallique (42) déposé au centre de ladite couche isolante et comportant une couche d'oxyde de tungstène (WO₃) qui se présente sous la forme d'une poudre agglomérée à structure poreuse, et
- deux électrodes (40) disposées au contact dudit élément sensible pour permettre de mesurer sa résistance électrique,
**caractérisé en ce que** ladite poudre d'oxyde de tungstène est constituée de particules de taille submicronique en forme d'écailles.

2. Capteur selon la revendication 1, **caractérisé en ce que** lesdites électrodes (40) sont intercalées entre la couche isolante (26) et l'élément sensible (42).

3. Capteur selon l'une des revendications 1 et 2, **caractérisé en ce que** lesdites électrodes (40) sont interdigitées.

4. Capteur selon l'une des revendications 1 à 3, **caractérisé en ce que** lesdites électrodes (40) sont en platine recouvert d'or.

5. Capteur selon l'une des revendications 1 à 4, **caractérisé en ce que** ledit cadre (10) est en silicium monocristallin.

6. Capteur selon l'une des revendications 1 à 5, **caractérisé en ce que** ladite couche diélectrique (14) est en oxynitrure.

7. Capteur selon l'une des revendications 1 à 6, **caractérisé en ce que** ladite couche conductrice (18) est en silicium polycristallin rendu conducteur par dopage.

8. Capteur selon l'une des revendications 1 à 7, **caractérisé en ce que** ladite couche isolante (26) est en oxyde ou nitrure de silicium.

9. Procédé de réalisation de l'élément sensible en oxyde de tungstène (42) du capteur selon la revendication 1, **caractérisé en ce qu'**il comporte les opérations successives de :
- dissolution de tungstate de sodium hydraté dans de l'eau distillée,
- passage à travers une colonne contenant une résine échangeuse de protons pour obtenir une solution d'acide tungstique,
- évaporation - concentration de cette solution afin d'obtenir, par précipitation, des particules d'oxyde de tungstène hydraté,
- dépôt desdites particules, sous forme d'une goutte, au centre de ladite couche isolante (26), et
- recuit du capteur.

## Claims

1. A gas chemical sensor with a metal oxide including:
- a frame (10) delimiting a central window (12),
- a dielectric layer (14) deposited on said frame and the portion of which facing its window forms a membrane (16),
- a conductive layer (18) forming a heating body, positioned on said membrane,
- an electrically insulating layer (26) covering said heating body,
- a metal oxide sensitive component (42) deposited at the centre of said insulating layer and including a tungsten oxide (WO₃) layer which has the form of an agglomerated powder with a porous structure, and
- two electrodes (40) positioned at the contact of said sensitive component so as to allow measurement of its electrical resistance,
**characterized in that** said tungsten oxide powder consists of particles of submicron size as flakes.

2. The sensor according to claim 1, **characterized in that** said electrodes (40) are inserted between the insulated layer (26) and the sensitive component (42).

3. The sensor according to any of claims 1 and 2, **characterized in that** said electrodes (40) are interdigitated.

4. The sensor according to any of claims 1 to 3, **characterized in that** said electrodes (40) are in gold-plated platinum.

5. The sensor according to any of claims 1 to 4, **characterized in that** said frame (10) is in monocrystalline silicon.

6. The sensor according to any of claims 1 to 5, **characterized in that** said dielectric layer (14) is in oxynitride.

7. The sensor according to any of claims 1 to 6, **characterized in that** said conductive layer (18) is in polycrystalline silicon made conductive by doping.

8. The sensor according to any of claims 1 to 7, **characterized in that** said insulated layer (26) is in silicon oxide or nitride.

9. A method for making the sensitive tungsten oxide component (42) of the sensor according to claim 1, **characterized in that** it includes the successive operations of:
- dissolving sodium tungstate hydrate in distilled water,
- passing it through a column containing a proton exchanger resin in order to obtain a solution of tungstic acid,
- evaporating - concentrating this solution in order to obtain tungsten oxide hydrate particles by precipitation,
- depositing said particles as a drop, at the centre of said insulating layer (26), and
- annealing the sensor.

## Patentansprüche

1. Chemischer Metalloxid-Gassensor, umfassend:
- einen Rahmen (10), der ein mittiges Fenster (12) begrenzt,
- eine dielektrische Schicht (14), die auf dem Rahmen aufgebracht ist und deren Abschnitt gegenüber seines Fensters eine Membran (18) bildet,
- eine leitende Schicht (18), die ein Heizelement bildet und auf der Membran aufgebracht ist,
- eine elektrisch isolierende Schicht (26), die das Heizelement bedeckt,
- ein sensibles Metalloxidelement (42), das in der Mitte der isolierenden Schicht aufgebracht ist und eine Wolframoxidschicht (WO₃) umfasst, die sich in Form eines aufgepressten Pulvers mit poröser Struktur darstellt, und
- zwei Elektroden (40), die im Kontakt mit dem sensiblen Element angeordnet sind, um die Messung seines elektrischen Widerstands zu erlauben,
**dadurch gekennzeichnet, dass** das Wolframoxidpulver von schuppenförmigen Partikeln in Submikrongröße gebildet wird.

2. Sensor nach Anspruch 1, **dadurch gekennzeichnet, dass** die Elektroden (40) zwischen der isolierenden Schicht (26) und dem sensiblen Element (42) angeordnet sind.

3. Sensor nach einem der Ansprüche 1 und 2, **dadurch gekennzeichnet, dass** die Elektroden (40) parallel geschaltet sind.

4. Sensor nach einem der Ansprüche 1 bis 3, **dadurch gekennzeichnet, dass** die Elektroden (40) aus vergoldetem Platin sind.

5. Sensor nach einem der Ansprüche 1 bis 4, **dadurch gekennzeichnet, dass** der Rahmen (10) aus monokristallinem Silizium ist.

6. Sensor nach einem der Ansprüche 1 bis 5, **dadurch gekennzeichnet, dass** die dielektrische Schicht (14) aus Oxynitrid ist.

7. Sensor nach einem der Ansprüche 1 bis 6, **dadurch gekennzeichnet, dass** die leitende Schicht (18) aus polykristallinem Silizium ist, die durch Dotieren leitend gemacht wurde.

8. Sensor nach einem der Ansprüche 1 bis 7, **dadurch gekennzeichnet, dass** die isolierende Schicht (26) aus Siliziumoxid oder -nitrid ist.

9. Verfahren zur Herstellung des sensiblen Elements aus Wolframoxid (42) des Sensors nach Anspruch 1, **dadurch gekennzeichnet, dass** es die aufeinanderfolgenden Schritte umfasst:
- Auflösen von hydriertem Natriumwolframat in destilliertem Wasser,
- Durchgang durch eine Säule, die ein protonenaustauschendes Harz enthält, um eine Wolframsäurelösung zu erhalten,
- Verdampfen - Konzentration dieser Lösung, um durch Ausfällen hydrierte Wolframoxidpartikel zu erhalten,
- Ablagern der Partikel in Tropfenform in der Mitte der isolierenden Schicht (26) und
- Glühen des Sensors.
